# EUROPEAN PATENT SPECIFICATION

(11) **EP 1 615 557 B1**
(45) Date of publication and mention of the grant of the patent: **19.09.2012**
(21) Application number: 04727649.8
(22) Date of filing: 15.04.2004
(51) Int. Cl.: A61B 5/06, A61K 49/18

(54) **METHOD AND APPARATUS FOR IMPROVED DETERMINATION OF SPATIAL NON-AGGLOMERATED MAGNETIC PARTICLE DISTRIBUTION IN AN AREA OF EXAMINATION**
VERFAHREN UND VORRICHTUNG ZUR VERBESSERTEN ERMITTLUNG DER RÄUMLICHEN VERTEILUNG NICHT AGGLOMERIERTER MAGNETISCHER PARTIKEL IN EINEM UNTERSUCHUNGSBEREICH
PROCEDE ET APPAREIL DE DETERMINATION AMELIOREE DE LA REPARTITION SPATIALE DE PARTICULES MAGNETIQUES NON AGREGEES DANS UNE ZONE D'EXAMEN

(30) Priority: 15.04.2003 EP 03101019
(43) Date of publication of application: 18.01.2006
(73) Proprietor: Philips Intellectual Property & Standards GmbH, 20099 Hamburg (DE); Koninklijke Philips Electronics N.V., 5621 BA Eindhoven (NL)
(72) Inventor: GLEICH, Bernhard, GmbH Weisshausstr. 2 52066 Aachen (DE)
(74) Representative: Volmer, Georg
(86) International application number: PCT/IB2004/050450
(87) International publication number: WO 2004/091398

(56) References cited:
- EP-A2- 1 304 542
- WO-A-91/02811
- US-A- 4 827 945
- US-A- 4 849 210
- US-A- 5 370 901
- KAISER R ET AL: "Magnetic properties of stable dispersions of subdomain magnetite particles" JOURNAL OF APPLIED PHYSICS USA, vol. 41, no. 3, 1970, pages 1064-1072, XP002295575 ISSN: 0021-8979 cited in the application

## Description

The present invention relates to a method and apparatus for improved determination of spatial magnetic particle distribution in an area of examination. In particular the invention relates to a method to prevent or reduce agglomeration of magnetic particles, in particular in a method to determine the spatial distribution of magnetic particles in an examination area of an object of examination. The invention further relates to an apparatus to determine the spatial distribution of magnetic particles comprising means to prevent agglomeration of magnetic particles.

Magnetic particles have proven themselves as contrast agents for in-vivo magnetic resonance imaging. They contribute significantly to improving proton relaxation times T₁ and T₂, and they help in this manner to obtain images with greater sharpness and clarity. An image with good sharpness is regularly obtained in nuclear spin tomography when the T₁ and/or T₂ of the tissue being imaged differ from the T₁ and/or T₂ of the background tissue. Paramagnetic materials are preferably used because of their ability to increase T₁, and superparamagnetic and ferromagnetic materials are used because of their ability to reduce T₂. T₁ is the spin-grid or longitudinal relaxation time and T₂ is the spin-spin or transverse relaxation time. Suitable contrast agents for nuclear spin tomography must be available in extremely stable formats so that specific working steps common to pharmaceutical production can be implemented, e.g. dialysis, filtration, centrifugation, storage of concentrates or sterilization.

Particle agglomeration or clumping contributes considerably to destabilization. To prevent clumping of magnetic particles, e.g. through Van der Waals forces or magnetic attraction, these particles are frequently coated. The solvent forms this coating in the so-called ferrofluids (Kaiser et al., J. Appl. Phys. 1970, 41 (3), page 1064). Attempts have also been made to coat iron oxide crystals with a polymer or surface active substances to prevent attractive forces that promote clumping. DE 3751918 T2 describes a four stage method for the production of a stable superparamagnetic fluid comprising bivalent and trivalent metal salts. Even though magnetic particles are obtained in DE 3751918 T2 that do not have a tendency to clump, the proposed manufacturing method is restricted to superparamagnetic fluids of bivalent and trivalent metal salts and cannot be easily transferred to other systems. The disadvantage remains that the proposed solution is specially tailored to the needs of nuclear spin tomography and cannot be generalized.

US 4,537, 861 discloses determination of the spatial distribution of magnetic particles, but does neither consider magnetization-dependent signals not imposition of a varying magnetic field.

The object of the present invention is to determine a magnetic measurement method not subject to the disadvantages in the prior art, more particularly, not negatively influenced by clumping or agglomeration phenomena of magnetic particles.

According to the invention there is provided a method is to prevent or reduce agglomeration of magnetic particles wherein the magnetic particles are exposed to a varying magnetic field. More in particular, there is provided a method to determine the spatial distribution of magnetic particles in an examination area of an object of examination with the following steps:
a) Generation of an imaging magnetic field with a spatial distribution of the magnetic field strength such that the area of examination consists of a first sub-area with lower magnetic field strength where the magnetization of the magnetic particles is not saturated and a second sub-area with a higher magnetic field strength where the magnetization of the magnetic particles is saturated,
b) Change of the spatial location of both sub-areas in the area of examination so that the magnetization of the particles changes locally,
c) Acquisition of signals that depend on the magnetization in the area of examination influenced by this change, and
d) Evaluation of said signals to obtain information about the spatial distribution of the signals in the area of examination, wherein the magnetic particles before or during the determining of the spatial distribution of the magnetic particles in the examination area are exposed to a varying magnetic field at least some of the time, more particularly periodically or continuously such as to reduce or prevent agglomeration of the magnetic particles.

In a preferred embodiment of the method according to the invention the gradient field in the area of examination has a varying magnetic field superimposed on the imaging magnetic field at least some of the time, more particularly periodically or continuously, more particularly, in at least parts of the first sub-area with lower magnetic field strength and wherein the strength of the varying magnetic field is sufficient to cancel out those attractive forces resulting in the clumping or agglomeration between neighboring particles in the area of examination.

The varying magnetic field can be specifically used to prevent agglomeration of magnetic particles in the area of examination in that the particles are prevented from aligning and arranging themselves with the mutual attractive forces.

It is provided here that the strength of the varying magnetic field can be specifically set to cancel out those attractive forces resulting in the clumping or agglomeration between neighboring particles in the area of examination.

When a varying magnetic field is applied in one direction, the magnetic particles align their magnetic moment in this direction and the magnetic forces between the particles may separate the particles if the angles are right. However, after the separation the particles can re-agglomerate by moving relative to each other such that a north pole of one magnetic particle comes closer to a south pole of another magnetic particle. Therefore, in a preferred embodiment of the method according to the invention it is provided that the varying magnetic field is present, more particularly, at an equal strength, in all three spatial components. The varying magnetic field in all three dimensions maybe applied simultaneously or in an alternating fashion. In this way,the magnetic particle is forced by the varying magnetic field within the area of examination into a movement which does not permit the forces acting between, e.g. neighboring magnetic particles, to effect an attachment.

In a preferred embodiment of the method, the magnetic particles have an average size or expansion of at least 30, more particularly at least 40 nm.

In another embodiment of the method according to the invention, the varying magnetic field can be applied, locally restricted, in the area of examination until the clumping or agglomeration of magnetic particles in this location is at least partially resolved. Basically, the method according to the invention is not only capable of preventing more or less freely moveable magnetic particles in an area of examination from agglomerating; it can also destroy previously formed agglomerates or clumps.

It is therefore possible to introduce the magnetic particles into the area of examination as free or de-agglomerated particles or in the form of particle agglomerates or clumps.

For both previously mentioned application areas of the method according to the invention, it has been shown to be particularly effective to use a varying magnetic field with a frequency in the range of approx. 1 kHz to 10 MHz, preferably 10 to 500 kHz. The frequency of the varying magnetic field can also be acquired as the frequency of square pulses or other wave shapes.

It can then be provided that the magnetic particle is a multi or mono-domain particle that can be reverse magnetized by Neel rotation and/or that the reverse magnetization is caused by Brownian rotation. Suitable magnetic particles are also hard or soft magnetic multi-domain particles.

The method according to the invention makes significant use of an arrangement as described in the German patent number DE 101 51 778. This refers also to the preferred embodiments of this arrangement in the above patent application.

The arrangement used in the invention generates a spatially inhomogenous magnetic field in the area of examination. In the first sub-area, the magnetic field is so weak that the magnetization of the particle deviates more or less strongly from the external field and is therefore not saturated. This first sub-area is preferentially a spatially coherent area; it can however also be a punctiform area, but also a line or a plane. In the second sub-area (i.e. the rest of the area of examination lying outside the first area), the magnetic field is sufficiently strong to hold the particle in a state of saturation. Magnetization is saturated when the magnetization has aligned almost all particles in approximately the direction of the external magnetic field so that with an increase in magnetic field, the magnetization in that area increases considerably less than in the first sub-area with a similar increase in magnetic field.

By changing the position of the two sub-areas within the area of examination, the (total) magnetization in the area of examination changes. If, therefore, the magnetization in the area of examination or the physical parameters influenced by this are measured, information can be derived about the spatial distribution of the magnetic particles in the area of examination.

To change the spatial position of both sub-areas in the area of examination or to change the magnetic field strength in the first sub-area, an e.g. magnetic field that is localized and/or changes over time can be generated. It is also provided that the signals induced in at least one coil by the change over time of the magnetization in the area of examination are acquired and evaluated to obtain information about the spatial distribution of magnetic particles in the area of examination. The biggest possible signals are achieved by changing the spatial position of both sub-areas as rapidly as possible. A coil, with which a magnetic field can be generated in the area of examination, can be used to acquire the signals. Preferably, at least one separate coil is used.

If the change in the spatial position of the sub-area is implemented, e.g. using a magnetic field changing over time, this can induce a similarly periodic signal in a coil. The acquisition of this signal may however be difficult as the signals generated in the area of examination and the magnetic field changing over time are simultaneously effective: it is therefore not possible to differentiate between the signals induced by the magnetic field and the signals induced by the change in magnetization in the area of examination. This can however be avoided in that a magnetic field changing over time acts on a first frequency band on the area of examination and that a second frequency band, which contains higher frequency components than the first frequency band, in the signal received from the coil is evaluated to obtain information about the spatial distribution of the magnetic particles. This exploits the fact that the frequency components of the second frequency band can only be created by a change in the magnetization in the area of examination due to the non-linearity of the magnetization characteristic curve. When the magnetic field changing over time has a sinusoidal periodic behavior, the first frequency band consists only of a single frequency component - the sinusoidal fundamental oscillation. In contrast, the second frequency band contains, in addition to this fundamental oscillation, higher harmonics (so-called harmonic waves) of the sinusoidal fundamental oscillation which can be used for evaluation.

A preferred arrangement for the method in the present invention is characterized in that the means for generating the magnetic field includes a gradient coil arrangement for generating a magnetic gradient field which reverses its direction in the first sub-area of the area of examination and evidences a zero passage. This magnetic field is - when the gradient coil arrangement, e.g. comprises two identical windings carrying opposing flows located on either side of the area of examination (Maxwell coil) - zero at a point on the winding axis and increases almost linearly on both sides of this point with opposite polarities. It is only with these particles located in the area around this field zero point where magnetization is not saturated. For particles outside this area, the magnetization is in a state of saturation.

Therefore an arrangement can be provided with means to generate a magnetic field changing over time and superimposed on the magnetic gradient field for the purpose of moving both sub-areas in the area of examination. The area generated by the gradient coil arrangement is therefore moved around the field zero point, i.e. the first sub-area, within the area of examination by the magnetic field changing over time. With appropriate changes over time and orientation of this magnetic field it is possible to move the field zero point throughout the entire area of examination.

The magnetization change resulting from the movement of the field zero point can be detected by an appropriate coil arrangement. The coil used to detect the signals generated in the area of examination can be a coil that is already used to generate the magnetic field in the area of examination. There are, however, advantages to using a separate coil for reception as this can be decoupled from the coil arrangement producing a magnetic field that changes over time. In addition, an improved signal/noise ratio can be achieved with a coil - and more so with several coils.

The amplitude of the signals induced in the coil arrangement increases the faster the position of the field zero point changes in the area of examination, i.e. the faster the magnetic field changing over time superimposed on the magnetic gradient field changes. It is however technically difficult to generate a magnetic field changing over time with sufficient amplitude to move the field zero point at the point of the area of examination or with sufficiently large change speed to generate signals with sufficient amplitude. Particularly suitable arrangements for this purpose comprise means to generate a first and at least a second magnetic field superimposed on the magnetic gradient field, where the first magnetic field moves slowly with high amplitude and the second magnetic field moves fast with low amplitude. This generates - preferably by two coil arrangements - two magnetic fields with different speeds and different amplitudes. Another advantage is that the field changes can be so fast (e.g. > 20 kHz) that they lie above the human limit of audibility. It can also be provided that both magnetic fields in the area of examination are generally aligned vertically to one another. This enables the movement of the field-free point within a two-dimensional area. This can be expanded to a three-dimensional area by another magnetic field comprising a component aligned vertically to the two magnetic fields. Another advantage is provided by an arrangement with a filter downstream of a coil arrangement which suppresses the signal components in a first frequency band in the signal induced by the coil arrangement and allows the signal components in a second frequency band, which contains higher frequency components than the first frequency components, to pass. This exploits the fact that the magnetization characteristic curve is non-linear in the area where the magnetization transitions from the non-saturated to the saturated state. This non-linearity has the effect that, e.g. a sinusoidal magnetic field over time with the frequency f generates, in the area of non-linearity, an induction changing over time with the frequency f (fundamental oscillation) and integer multiples of the frequency f (harmonic waves or higher harmonics). The evaluation of the higher harmonics has the advantage that the fundamental oscillation of the magnetic field used to move the field-free point does not have any influence on the evaluation.

According to the present invention, it is provided that the magnetic particles become saturated when an external magnetic field is applied, especially one with a strength of circa 100 mT or less. Naturally, larger saturation field strengths are also suitable for the method according to the invention.

Suitable magnetic field strengths for many applications are already circa 10 mT or less. This strength would already be sufficient for many tissue or organ examinations. But it is also possible to achieve good measurement results with field strengths in the area of 1 mT or less, or circa 0.1 mT or less. For example, concentration data, temperature, pressure or pH values can be determined with high accuracy and resolution with magnetic fields of circa 10 mT or less, circa 1 mT or less and circa 0.1 mT or less. It is noted that strictly speaking, magnetic field strength is expressed in H (A/m). However, in the present application, when reference is made to magnetic field strength, B-fields are meant. A magnetic fields B of 1 mT as described above corresponds to an H field of 1 mT/µ₀ = 0.8 kA/m, that is the equivalent H field that would produce a B field of 1 mT in vacuum.

In the sense of the present invention, an external magnetic field, where the magnetic particles become or are saturated, means a magnetic field where circa half the saturation magnetization is achieved.

Suitable magnetic particles here are those that can reach saturation with a sufficiently small magnetic field. A necessary requirement for this is that the magnetic particles have a minimum size or a minimum dipole moment. The term magnetic particle in the sense of the present invention also comprises particles that can be magnetized.

Suitable magnetic particles favorably have dimensions that are small compared to the size of the voxel whose magnetization is to be determined by the method according to the invention. In addition, the magnetization of the particles should preferably reach saturation at the lowest possible field strengths of the magnetic field. The lower the field strength required for this is, the higher the spatial resolution capacity or the weaker the (external) magnetic field being generated in the area of examination can be. In addition, the magnetic particles must have the highest possible dipole moment or a high saturation induction so that the change in magnetization produces the largest possible output signals. It is also important for the particles not to be toxic if the method is to be used for medical examinations.

A preferred embodiment of the present method according to the invention provides that the magnetic particle is a mono-domain particle that can be reverse magnetized by Neel rotation and/or that the reverse magnetization is caused by Brownian rotation.

Suitable magnetic mono-domain particles are preferably dimensioned so that only a single magnetic domain (the mono-domain) can be formed in them or Weiß areas are not present. Suitable particle sizes in a specially preferred embodiment of the present invention lie in the range between 20 nm to ca. 800 nm, where the upper limit is also dependent on the material used. Preferably, magnetite (Fe₃O₄), maghemite (γ-e₂O₃) and/or non-stoichiometric magnetic iron oxides are used as mono-domain particles.

In general it is advantageous, especially when a rapid reverse magnetization based on Neel rotation is required, for the mono-domain particles to evidence a low effective anisotrophy. Effective anisotrophy means the anisotrophy resulting from the form anisotrophy and the average crystal anisotrophy. In the above-mentioned case a change in magnetization direction does not require the particle to be turned. Alternatively, mono-domain particles with high effective anisotropy can be used when it is desired that the reverse magnetization, when applying an external magnetic field, is implemented by Brownian or geometric rotation.

An alternative embodiment of the present method according to the invention provides that the magnetic particle can be a hard or soft magnetic multi-domain particle. These multi-domain particles are usually larger magnetic particles in which a number of magnetic domains can be formed. Such multi-domain particles suitably have a low saturation induction.

Hard magnetic multi-domain particles generally have the same magnetic properties as mono-domain particles with higher effective anisotrophy. Soft magnetic multi-domain particles with low saturation magnetization have the advantage that they can be shaped into any form for use in the present method according to the invention. If they have an asymmetrical external form, they are then particularly suitable for local viscosity measurements in the area of examination. Soft magnetic multi-domain particles with high saturation magnetization must preferably be designed so that the demagnetizing factor becomes small. Both symmetrical and asymmetrical forms can be considered here. For example, a soft magnetic material with high saturation magnetization can be applied as a thin coating on a ball or cube that is not magnetizable. Soft magnetic multi-domain particles with high saturation magnetization that have an asymmetrical form, e.g. in the form of flakes or needles, can also be used for viscosity measurements.

Therefore, mono-domain particles, where reverse magnetization occurs via Neel and Brownian rotation, are particularly suitable for local viscosity measurements in the area of examination as are soft magnetic multi-domain particles with small or large saturation magnetization that have an asymmetrical external form.

As described above, the magnetic particles also comprise such particles that consist of a non-magnetic core and a coating of a magnetic material. Therefore this comprises in general all magnetic particles that have a low effective anisotrophy and those that have a high effective anisotrophy. A high coercive force H_{c} is necessary in semi-hard and, especially, hard magnets in order to bring the magnetization to zero. Suitably hard magnetic materials comprise Al-Ni, Al-Ni-Co and Fe-Co-V alloys as well as barium ferrite (BaO 6xFe₂O₃).

In the method according to the invention the field strength of the varying magnetic field is typically at least two times, preferably is three times higher than the field strength of the imaging magnetic field. The field strength of the varying magnetic field depends on the size and nature of the magnetic particles. According to one embodiment of the method of the invention wherein the magnetic particles are monodomain particles, the field strength of the varying magnetic field is preferably at least 30, preferably at least 50 mTesla. In another embodiment wherein the magnetic particles comprise a nonmagnetic core covered with a magnetic coating, the field strength of the varying magnetic field is at least five mTesla.

The field strength and the frequency of the varying magnetic field must on the one hand be high enough to prevent the clumping or agglomeration of the magnetic particles, but on the other hand must be not to high to not damage the object of examination. In case the object of the examination is a living organism, it must be prevented that the varying magnetic field heats up the organism. In the preferred case where the examination area is in a living organism of about human size, it is preferred that the varying magnetic field has a power input of less than 500 Watt, preferably less than 300 W. These values are avarage values over about 10 seconds so actual peak values may be much higher. Preferably, the field strength of the varying magnetic field is less than 20, preferably less than 10 mTesla. In case a very high power input is required, for example to de- agglomerates seriously agglomerated magnetic particles, it is preferred that the varying magnetic field has a power of at least 300 to 500W and is applied in intermittent pulses such that the average power input is less than 300 to 500 W. For example, when a continuous wave with 20 mT at 100 kHz is applied, a patient would still be below the heat limit. Higher amplitudes than that may be applied, but then it may only applied in short bursts.

In the method according to the invention the magnetic particles are in a liquid medium in the examination area and the frequency of the varying magnetic field is chosen in view of the viscosity of said medium. For example, in a preferred application the medium surrounding the magnetic particles is blood and the frequency of the varying magnetic field is preferably between 0.5 and 1.5MHz, preferably 0.7 and 1.3 MHz.

In a preferred embodiment of the method according to the invention the varying magnetic field is applied as one or more pulses having an amplitude reducing to zero in a time sufficient to increase the distance between agglomerated particles sufficiently to prevent re-agglomeration. The amplitude at the onset of the pulse can be chosen very high to effectively separate agglomerated magnetic particles. Once the magnetic particles are partly separated a lower amplitude is required to further move the magnetic particles apart. The frequency of the varying magnetic fields pulse this chosen in view of the re-agglomeration time scale, which depends on the viscosity of the medium surrounding the magnetic particles.

As described above particle agglomeration causes a reduced response of the magnetic particles to the imaging magnetic field. The varying magnetic field can be applied in different ways to the magnetic particles to prevent agglomeration or to deagglomerate and separate agglomerated particles. For example, the varying magnetic field is applied to magnetic particles shortly before administering to the examination area. In this embodiment the magnetic particles are administered to the examination area in a de-agglomerated state which results in better imaging properties. The advantage of this is that during the examination of the examination area with the imaging method according to the invention the varying magnetic field can be used at a lower magnetic field strength or can even be omitted.

In a special embodiment of the method according to the invention the magnetic particles are administered to the examination area in an agglomerated state and the varying magnetic field is applied locally to only a part of the examination area. In this special embodiment it is possible to introduce responsive magnetic particles in only a selective part of the examination area and to subsequently follow the development in time and space of the magnetic particles from that selected area. For example, it is possible to locally switch on in an active responsive state a part of the administered magnetic particles for example in a blood vessel and see how these particles move with the blood stream in the organism.

In another embodiment of the invention the varying magnetic field and the imaging magnetic field are applied in an alternating fashion. This is particularly preferred in a situation where the frequency of the varying magnetic field is close to the frequency of the imaging magnetic field, in particular where the frequency of the varying magnetic field is 0.8 to 1.2 times the frequency of the imaging magnetic field.

The invention further relates to an aparatus to determine the spatial distribution of magnetic particles in an area of examination in an object of examination comprising:
means to generate a magnetic field with a spatial distribution of the magnetic field strength such that the area of examination consists of a first sub-area with lower magnetic field strength and a second sub-area with a higher magnetic field strength,
means to change the spatial location of both sub-areas in the area of examination so that the magnetization of the particles changes locally,
means for the acquisition of signals that depend on the magnetization in the area of examination influenced by this change, and
means for the evaluation of said signals to obtain information about the spatial distribution of the signals in the area of examination,
means to superimpose in the area of examination, more particularly, in at least parts of the first sub-area with lower magnetic field strengths, a varying magnetic field at least some of the time, more particularly periodically or continuously.

The advantage of said aparatus according to the invention is that the aparatus has an improved imaging performance because clumping can be effectively prevented and higher concentrations of the magnetic particles are feasible. Further, it is not longer required that magnetic particles or compositions containing said magnetic particles are prevented from clumping, due to which a wider range of magnetic particles can be used in the imaging procedure. A further advantage of the aparatus is, as described above, that it makes possible a further technique to trace in time and space the trajectory of magnetic particles and the examination area.

In general the magnetic particles are chosen such that good magnetic particle images, in particular a good resolution can be obtained in a given field gradient. In German patent number DE 101 51778 a magnetic particle imaging method is described. It is generally described that magnetic mono-domain particles having a size between 20 and 800 nanometres or a glass beat coated with a magnetic coating can be used in this method. However, in order to achieve a good magnetic imaging contrast and resolution at relatively low magnetic field gradients, improved magnetic particle compositions are highly desirable. Preferably, the magnetic particles have a magnetization curve having a step change, the step change being wherein the magnetization change, as measured in an aqueous suspension, in a first field strength window of magnitude delta around the inflection point of said step change is at least a factor 3 higher than the magnetization change in the field strength windows of magnitude delta below and/or in the field strength windows of magnitude delta above the first field strength window, wherein delta is less than 2000 microtesla, preferably less than 1000 microtesla, and wherein the time in which the magnetisation step change is completed in the first delta window is less than 0.01 seconds, preferably less than 0.005 sec, more preferably less than 0.001, most preferably less than 0.0005 seconds. Such magnetic particles are particularly suitable for magnetic particle imaging, in particular for obtaining a good resolution of the image. It is further preferred, that the magnetic particle composition has a magnetisation curve, wherein the step change is at least 10%, preferably at least 20 %, more preferably at least 30 % and most preferably at least 50% of the total magnetisation of the particle composition as measured at an external magnetisation field of 1 Tesla. It is further preferred, that the magnetization change in the first field strength window of magnitude delta around the inflection point of said step change is at least a factor 4, preferably at least a factor 5 higher than the magnetization change in the field strength windows of magnitude delta below or in the field strength windows of magnitude delta above the first field strength window.

The magnetic particle composition is particularly useful for use in a magnetic particle imaging technique. The particles show good spatial resolution at relatively low field strength gradients. Further, the magnetic particle composition allows for a relatively high scanning speed for examining a large examination area. For example, for application in medical magnetic particle imaging, where the step change occurs preferably at a delta value below 1000 microTesla, the particle composition has a resolution value better than between 0.1 and 10 mm at magnetic field strength gradients between 10 and 0.1 T/m. With the magnetic particle imaging technique using the magnetic particle compositions extremely good resolution can be obtained, for example in a range from 0.1 to 10 micrometres in applications, where are very high magnetic field is gradients can be achieved, for example in microscopy. It is noted that strictly speaking, magnetic field strength is expressed in H (A/m). However, in the present application, when reference is made to magnetic field strength, B-fields are meant. A magnetic fields B of 2000 µT as described above corresponds to an H field of 2 mT/µ0 = 1.6 kA/m, that is the equivalent H field that would produce a B field of 2 mT in vacuum.

Preferably, the optical contrast composition and the method according to the invention as described above comprise magnetic particles that meet the specified step change requirements of the magnetic particle composition as described above.

A method for measuring the magnetisation curve and the required step change is as follows. A sample of a magnetic particle composition is suspended in water, optionally with the help of a simple detergent. To prevent clumping and/or to deagglomerate the magnetic particles an ultrasound treatment possible can be used. The concentration of the magnetic particle composition is less than 0.01gr core mass per liter of solvent. With core mass is meant the mass of the magnetic material in the magnetic particle composition. The suspension is brought into a fast magnetometer. (i.e. a device that measures the magnetization of the sample while an external field is applied). Suitable fast magnetometers are known to the expert. The magnetometer is equipped with means allowing to produce an external field at the sample position in at least two orthogonal directions simultaneously, i.e. to produce any magnetic field below a given maximum amplitude and a given maximum speed of change. The magnetisation is measured also in at least two orthogonal directions in the same plane.

First the saturation magnetisation is measured. For this, a magnetic field of about one Tesla is applied in one direction and the magnitude of magnetization is measured after at least 10 seconds. Then the measurement sequences for determining the step change starts. The sequence starts with choosing a field vector with an external field magnitude below 20mT. This field is applied for at most 100 seconds. Then a second direction is chosen. This direction defines the scalar values of the field H and the magnetization M. The field is rapidly changed, preferably less than 1 millisecond, so that it lies now in -H direction with some magnitude below 20 mT. Then the field is changed from -H to +H e.g. in a linear way and the (now scalar i.e. projected) magnetization is recorded. The magnetization curve is recorded in less than 0.01s but longer than 1µs. Where the magnetisation curve shows a step change, a first window of size delta is positioned centrally on the inflection point of the magnetisation step change. Similarly, a window of size delta is positioned below and above the first window, and the required step change is evaluated by determining the change in magnetisation in each of the windows.

Whether or not a given magnetic particle composition has the required step change depends in a complicated way on many variables, for example of the size of the particles, the particle size distribution, the shape of the particles, the damping constant for Neel rotation, the type of magnetic material, the crystallinity and the stochiometry of the composition of the magnetic material. It has been found that it is particularly important that the particle size distribution of the particle composition is narrow. Preferably, the magnetic particle composition has a narrow particle size distribution wherein at least 50 weight % of the particles have a particle size between plus or minus 50%, preferably 25%, more preferably 10% of the average particle size. Preferably, the amount of particles within the specified windows, is at least 70 wt %, preferably at least 80 wt %, and most preferably at least 90 wt %. Particularly good results are obtained with mono-domain particles have a low magnetic anisotropy with a field needed for inducing Neel rotation of substantially below 10mT, preferably below 5 mT, more preferably below 2 mT. Preferably, the magnetic particles are mono-domain particles having an average particle size between 20 and 80 nanometres, more preferably between 25 and 70 nanometres, must preferably between 30 and 60 nanometres, wherein at least 50, preferably at least 60, more preferably at least 70 weight % of the particles have a particle size between the average particle size plus or minus 10 nanometre.

Alternatively, the magnetic particle can be a multi-domain particle having substantially a needle shape having a demagnetisation factor of less than 0.001. This magnetic particle composition is particularly useful in non-medical applications where the needles shape is not a disadvantage. The magnetic particle composition can also comprise magnetic particles comprising a non-magnetic core covered with a magnetic coating material, wherein the thickness of the coating is between 5 and 80 nanometres and wherein the demagnetisation factor is less than 0.01 and a diameter below 300µm. It is thereby advantageous to have a small particle size distribution as described above. The physical parameters of the magnetic particles are preferably chosen to meet the step change requirement as described above for achieving good imaging properties.

The magnetic particle composition can be manufactured by first forming magnetic particles, for example by precipitation, for example by contacting a solution comprising ferrous and ferric ions with a solution comprising sodium hydroxide as described above. In principle, a known precipitation process can be used. It is also possible to grind the particles from bulk material, for example using a high speed ball mill. The essential next step for obtaining a good magnetic particle composition is the selection and separation of the particles. The first step is to perform a size selection process by filtering and/or centrifuge methods. The next step is to perform a selection process based on the magnetic properties of the particles, for example, using oscillating magnetic gradient fields.

The present invention is based on the surprising recognition that it is possible to use magnetic particles in a magnetic imaging process, even in high concentrations, without fearing imaging disturbances by clumping or agglomeration phenomena. It is also surprising that a particle agglomeration can be prevented without having to coat every individual particle. Instead, the method according to the invention enables particle coating to be completely avoided. It is also an advantage that clumping or agglomeration phenomena can be specifically used, i.e. the agglomeration of magnetic particles can be specifically used or permitted and subsequently resolved by applying the invention process. It is therefore possible to both prevent magnetic particles from changing to an agglomerated state and to return (previously) clumped or agglomerated particles to a freely moveable state. Practically, the measures necessary for the deagglomeration or prevention of agglomeration can be superimposed on the measurement steps required for magnetic imaging.

The characteristics of the invention described above and in the claims can be used both individually and in any combination for the implementation of the invention in its various embodiments.

## Claims

1. A method to determine the spatial distribution of magnetic particles in an examination area of an object of examination with the following steps:
a) Generation of an imaging magnetic field with a spatial distribution of the magnetic field strength such that the area of examination consists of a first sub-area with lower magnetic field strength where the magnetization of the magnetic particles is not saturated and a second sub-area with a higher magnetic field strength where the magnetization of the magnetic particles is saturated,
b) Change of the spatial location of both sub-areas in the area of examination so that the magnetization of the particles changes locally,
c) Acquisition of signals that depend on the magnetization in the area of examination influenced by this change, and
d) Evaluation of said signals to obtain information about the spatial distribution of the signals in the area of examination, wherein the magnetic particles before or during the determining of the spatial distribution of the magnetic particles in the examination area are exposed to a varying magnetic field superimposed on the imaging magnetic field at least some of the time, more particularly periodically or continuously such as to reduce or prevent agglomeration of the magnetic particles.

2. A method according to claim 1, **characterized by** a gradient field in the area of examination that has a varying magnetic field superimposed on the imaging magnetic field at least some of the time in at least parts of the first sub-area with lower magnetic field strengths.

3. A method as claimed in claim 1, **characterized in that** the strength of the varying magnetic field is sufficient to cancel out those attractive forces resulting in the clumping or agglomeration between neighboring particles in the area of examination

4. A method as claimed in claim 1, **characterized in that** the varying magnetic field is applied at an equal strength in all three spatial components.

5. A method as claimed in any one of the preceding claims, **characterized in that** the particles have an average size or expansion of at least 30 µm, more particularly, at least 40 nm.

6. A method as claimed in any one of the preceding claims **characterized in that** the varying magnetic field is applied, locally restricted, in the area of examination until the clumping or agglomeration of magnetic particles in this location is at least partially resolved.

7. A method as claimed in any one of the preceding claims, **characterized in that** a varying magnetic field with a frequency in the range of approx. 1 kHz to 10 MHz, preferably 10 to 500 kHz is used.

8. A method as claimed in any one of the preceding claims, **characterised in that** the field strength of the varying magnetic field is at least two times higher than the field strength of the imaging magnetic field.

9. A method as claimed in any one of the preceding claims, wherein the magnetic particles are monodomain particles and wherein the field strength of the varying magnetic field is at least 30 µmTesla, preferably at least 50 mTesla.

10. A method as claimed in any one of claims 1 to 8, wherein the magnetic particles comprise a nonmagnetic core covered with a magnetic coating and wherein the field strength of the varying magnetic field is at least five mTesla.

11. A method as claimed in any one of the preceding claims, wherein the varying magnetic field has a power of at least 500 W and is applied in intermittent pulses such that the average power input is less than 500 W.

12. A method according to any one of the preceding claims, wherein the varying magnetic field is applied as one or more pulses having an amplitude reducing to zero in a time sufficient to increase the distance between agglomerated particles sufficiently to prevent re-agglomeration.

13. A method according to any one of the preceding claims, wherein the magnetic particles are in a liquid medium in the examination area and the frequency of the varying magnetic field is chosen in view of the viscosity of said medium.

14. A method according to claim 13, wherein the medium surrounding the magnetic particles is blood and the frequency of the varying magnetic field is between 0.7 and 1.3 MHz.

15. A method according to any one of claims 1 to 14, wherein the varying magnetic field is applied to magnetic particles shortly before administering to the examination area.

16. A method according to any one of claim 1 to 14, wherein magnetic particles are administered to the examination area in an agglomerated state and wherein in only a part of the examination area the particles are de-agglomerated.

17. A method according to any one of claims 1 to 16, wherein the frequency of the varying magnetic field is close to the frequency of the imaging magnetic field and wherein the exposure to the varying magnetic field and the exposure to the imaging magnetic field alternates.

18. A method according to claim 17, wherein the frequency of the varying magnetic field is 0.8 to 1.2 times the frequency of the imaging magnetic field.

19. A method as claimed in any one of the preceding claims, **characterized in that** the magnetic particle is a multi or mono-domain particle that is reversely magnetizable by Neel rotation and/or Brownian rotation.

20. A method as claimed in any one of the preceding claims, **characterized in that** the magnetic particle is a hard or soft magnetic multi-domain particle.

21. An aparatus to determine the spatial distribution of magnetic particles in an area of examination in an object of examination comprising:
a) means to generate a magnetic field with a spatial distribution of the magnetic field strength such that the area of examination consists of a first sub-area with lower magnetic field strength where the magnetization of the magnetic particles is not saturated and a second sub-area with a higher magnetic field strength where the magnetization of the magnetic particles is saturated,
b) means to change the spatial location of both sub-areas in the area of examination so that the magnetization of the particles changes locally,
c) means for the acquisition of signals that depend on the magnetization in the area of examination influenced by this change,
d) means for the evaluation of said signals to obtain information about the spatial distribution of the signals in the area of examination and
e) means to impose in the area of examination, more particularly, in at least parts of the first sub-area with lower magnetic field strengths, a varying magnetic field at least some of the time, more particularly periodically or continuously such as to reduce or prevent agglomeration of the magnetic particles.

## Patentansprüche

1. Verfahren zur Ermittlung der räumlichen Verteilung magnetischer Partikel in einem Untersuchungsbereich eines Untersuchungsobjekts, wobei das Verfahren die folgenden Schritte umfasst, wonach:
a) ein abbildendes Magnetfeld mit einer räumlichen Verteilung der Magnetfeldstärke erzeugt wird, so dass der Untersuchungsbereich aus einem ersten Teilbereich mit einer geringeren Magnetfeldstärke, in dem die Magnetisierung der magnetischen Partikel nicht gesättigt ist, sowie einem zweiten Teilbereich mit einer höheren Magnetfeldstärke, in dem die Magnetisierung der magnetischen Partikel gesättigt ist, besteht,
b) die räumliche Position beider Teilbereiche in dem Untersuchungsbereich verändert wird, so dass sich die Magnetisierung der Partikel lokal verändert,
c) Signale erfasst werden, die von der Magnetisierung in dem Untersuchungsbereich, die von dieser Änderung beeinflusst wird, abhängig sind, und
d) die Signale ausgewertet werden, um Informationen über die räumliche Verteilung der Signale in dem Untersuchungsbereich zu erhalten, wobei die magnetischen Partikel vor oder während des Ermittelns der räumlichen Verteilung der magnetischen Partikel in dem Untersuchungsbereich einem variierenden Magnetfeld, das dem abbildenden Magnetfeld zumindest manchmal, vorzugsweise periodisch oder kontinuierlich, überlagert ist, ausgesetzt werden, um eine Agglomeration der magnetischen Partikel zu reduzieren oder zu verhindern.

2. Verfahren nach Anspruch 1, **gekennzeichnet durch** ein Gradientenfeld in dem Untersuchungsbereich, das ein variierendes Magnetfeld aufweist, das dem abbildenden Magnetfeld zumindest manchmal in zumindest Teilen des ersten Teilbereichs mit geringeren Magnetfeldstärken überlagert ist.

3. Verfahren nach Anspruch 1, **dadurch gekennzeichnet, dass** die Stärke des variierenden Magnetfelds ausreicht, um solche Anziehungskräfte, die in der Verklumpung oder Agglomeration zwischen benachbarten Partikeln in dem Untersuchungsbereich resultieren, aufzuheben.

4. Verfahren nach Anspruch 1, **dadurch gekennzeichnet, dass** das variierende Magnetfeld in einer gleichen Stärke in allen drei räumlichen Komponenten angelegt wird.

5. Verfahren nach einem der vorangegangenen Ansprüche, **dadurch gekennzeichnet, dass** die Partikel eine durchschnittliche Größe oder Expansion von mindestens 30 nm, vorzugsweise mindestens 40 nm, aufweisen.

6. Verfahren nach einem der vorangegangenen Ansprüche, **dadurch gekennzeichnet, dass** das variierende Magnetfeld in dem Untersuchungsbereich lokal beschränkt angelegt wird, bis die Verklumpung oder Agglomeration von magnetischen Partikeln an dieser Stelle zumindest zum Teil beseitigt ist.

7. Verfahren nach einem der vorangegangenen Ansprüche, **dadurch gekennzeichnet, dass** ein variierendes Magnetfeld mit einer Frequenz in dem Bereich von ca. 1 kHz bis 10 MHz, vorzugsweise 10 bis 500 kHz, verwendet wird.

8. Verfahren nach einem der vorangegangenen Ansprüche, **dadurch gekennzeichnet, dass** die Feldstärke des variierenden Magnetfelds mindestens zweimal höher als die Feldstärke des abbildenden Magnetfelds ist.

9. Verfahren nach einem der vorangegangenen Ansprüche, wobei die magnetischen Partikel Monodomainen-Partikel sind, und wobei die Feldstärke des variierenden Magnetfelds mindestens 30 m Tesla, vorzugsweise mindestens 50 m Tesla, beträgt.

10. Verfahren nach einem der Ansprüche 1 bis 8, wobei die magnetischen Partikel einen nicht-magnetischen Kern umfassen, der mit einer magnetischen Beschichtung versehen ist, und wobei die Feldstärke des variierenden Magnetfelds mindestens fünf m Tesla beträgt.

11. Verfahren nach einem der vorangegangenen Ansprüche, wobei das variierende Magnetfeld eine Leistung von mindestens 500 W aufweist und in intermittierenden Impulsen so angelegt wird, dass die durchschnittliche Leistungsaufnahme geringer als 500 W ist.

12. Verfahren nach einem der vorangegangenen Ansprüche, wobei das variierende Magnetfeld als ein oder mehrere Impulse mit einer Amplitude angelegt wird, die in einem Zeitraum, der ausreicht, um den Abstand zwischen agglomerierten Partikeln so zu erhöhen, dass eine Re-Agglomeration verhindert wird, auf Null zurückgeht.

13. Verfahren nach einem der vorangegangenen Ansprüche, wobei sich die magnetischen Partikel in einem flüssigen Medium in dem Untersuchungsbereich befinden und die Frequenz des variierenden Magnetfelds mit Blick auf die Viskosität des Mediums gewählt wird.

14. Verfahren nach Anspruch 13, wobei das die magnetischen Teilchen umgebende Medium Blut ist und die Frequenz des variierenden Magnetfelds zwischen 0,7 und 1,3 MHz liegt.

15. Verfahren nach einem der Ansprüche 1 bis 14, wobei das variierende Magnetfeld kurz vor Anwenden auf den Untersuchungsbereich an magnetische Partikel angelegt wird.

16. Verfahren nach einem der Ansprüche 1 bis 14, wobei magnetische Partikel dem Untersuchungsbereich in einem agglomerierten Zustand zugeführt werden, und wobei in nur einem Teil des Untersuchungsbereichs die Partikel de-agglomeriert werden.

17. Verfahren nach einem der Ansprüche 1 bis 16, wobei die Frequenz des variierenden Magnetfelds nah an der Frequenz des abbildenden Magnetfelds liegt, und wobei die Berührung mit dem variierenden Magnetfeld und die Berührung mit dem abbildenden Magnetfeld alternieren.

18. Verfahren nach Anspruch 17, wobei die Frequenz des variierenden Magnetfelds 0,8 bis 1,2 Mal die Frequenz des abbildenden Magnetfelds ausmacht.

19. Verfahren nach einem der vorangegangenen Ansprüche, **dadurch gekennzeichnet, dass** das magnetische Partikel ein Multi- oder Mono-Domainen-Partikel ist, das durch Neel-Rotation und/oder Brownsche Rotation umgekehrt magnetisierbar ist.

20. Verfahren nach einem der vorangegangenen Ansprüche, **dadurch gekennzeichnet, dass** das magnetische Partikel ein hartes oder weiches magnetisches Multi-Domainen-Partikel ist.

21. Vorrichtung zur Ermittlung der räumlichen Verteilung magnetischer Partikel in einem Untersuchungsbereich eines Untersuchungsobjekts, mit:
a) Mitteln zum Erzeugen eines Magnetfelds mit einer räumlichen Verteilung der Magnetfeldstärke, so dass der Untersuchungsbereich aus einem ersten Teilbereich mit einer geringeren Magnetfeldstärke, in dem die Magnetisierung der magnetischen Partikel nicht gesättigt ist, sowie einem zweiten Teilbereich mit einer höheren Magnetfeldstärke, in dem die Magnetisierung der magnetischen Partikel gesättigt ist, besteht,
b) Mitteln zur Veränderung der räumlichen Position beider Teilbereiche in dem Untersuchungsbereich, so dass sich die Magnetisierung der Partikel lokal verändert,
c) Mitteln zur Erfassung von Signalen, die von der Magnetisierung in dem Untersuchungsbereich, die von dieser Änderung beeinflusst wird, abhängig sind,
d) Mitteln zur Auswertung der Signale, um Informationen über die räumliche Verteilung der Signale in dem Untersuchungsbereich zu erhalten, und
e) Mitteln, um in dem Untersuchungsbereich, vorzugsweise in zumindest Teilen des ersten Teilbereichs mit geringeren Magnetfeldstärken, ein variierendes Magnetfeld zumindest manchmal, vorzugsweise periodisch oder kontinuierlich, anzulegen, um eine Agglomeration der magnetischen Partikel zu reduzieren oder zu verhindern.

## Revendications

1. Procédé pour déterminer la distribution spatiale de particules magnétiques dans une zone d'examen d'un objet d'examen avec les étapes suivantes :
a) la génération d'un champ magnétique d'imagerie avec une distribution spatiale de l'intensité de champ magnétique de sorte que la zone d'examen soit constituée d'une première sous-zone avec une intensité inférieure de champ magnétique où la magnétisation des particules magnétiques n'est pas saturée et une seconde sous-zone avec une intensité supérieure de champ magnétique où la magnétisation des particules magnétiques est saturée,
b) le changement de la localisation spatiale des deux sous-zones dans la zone d'examen de sorte que la magnétisation des particules change localement,
c) l'acquisition de signaux qui dépendent de la magnétisation dans la zone d'examen influencée par ce changement, et
d) l'évaluation desdits signaux pour obtenir des informations sur la distribution spatiale des signaux dans la zone d'examen, dans lequel les particules magnétiques avant ou durant la détermination de la distribution spatiale des particules magnétiques dans la zone d'examen sont exposées à un champ magnétique varié superposé sur le champ magnétique d'imagerie pendant au moins une certaine partie du temps, plus particulièrement périodiquement ou en continu afin de réduire ou empêcher l'agglomération des particules magnétiques.

2. Procédé selon la revendication 1, **caractérisé par** un champ gradient dans la zone d'examen qui possède un champ magnétique varié superposé sur le champ magnétique d'imagerie pendant au moins une certaine partie du temps dans au moins des parties de la première sous-zone avec des intensités inférieures de champ magnétique.

3. Procédé selon la revendication 1, **caractérisé en ce que** l'intensité du champ magnétique varié est suffisante pour annuler les forces d'attraction entraînant l'agglutination ou agglomération entre des particules voisines dans la zone d'examen.

4. Procédé selon la revendication 1, **caractérisé en ce que** le champ magnétique varié est appliqué à une intensité égale dans toutes les trois composantes spatiales.

5. Procédé selon une quelconque des revendications précédentes, **caractérisé en ce que** les particules possèdent une taille ou expansion moyenne d'au moins 30 nm, plus particulièrement, au moins 40 nm.

6. Procédé selon une quelconque des revendications précédentes, **caractérisé en ce que** le champ magnétique varié est appliqué, limité localement, dans la zone d'examen jusqu'à ce que l'agglutination ou agglomération de particules magnétiques dans cet emplacement soit au moins partiellement résolue.

7. Procédé selon une quelconque des revendications précédentes, **caractérisé en ce qu'**un champ magnétique varié avec une fréquence dans la plage d'approximativement 1 kHz à 10 MHz, de préférence 10 à 500 kHz est utilisé.

8. Procédé selon une quelconque des revendications précédentes, **caractérisé en ce que** l'intensité de champ du champ magnétique varié est au moins deux fois plus importante que l'intensité de champ du champ magnétique d'imagerie.

9. Procédé selon une quelconque des revendications précédentes, dans lequel les particules magnétiques sont des particules mono-domaine et dans lequel l'intensité de champ du champ magnétique varié est au moins 30 mTesla, de préférence au moins 50 mTesla.

10. Procédé selon une quelconque selon les revendications 1 à 8, dans lequel les particules magnétiques comprennent un noyau non magnétique recouvert avec un revêtement magnétique et dans lequel l'intensité de champ du champ magnétique varié est au moins cinq mTesla.

11. Procédé selon une quelconque des revendications précédentes, dans lequel le champ magnétique varié possède une puissance d'au moins 500 W et est appliqué dans des impulsions intermittentes de sorte que l'entrée de puissance moyenne soit inférieure à 500 W.

12. Procédé selon une quelconque des revendications précédentes, dans lequel le champ magnétique varié est appliqué sous forme d'une ou de plusieurs impulsions possédant une amplitude diminuant jusqu'à zéro dans un temps suffisant pour augmenter la distance entre des particules agglomérées suffisamment pour empêcher la ré-agglomération.

13. Procédé selon une quelconque des revendications précédentes, dans lequel les particules magnétiques sont dans un milieu liquide dans la zone d'examen et la fréquence du champ magnétique varié est choisie au vu de la viscosité dudit milieu.

14. Procédé selon la revendication 13, dans lequel le milieu entourant les particules magnétiques est du sang et la fréquence du champ magnétique varié est entre 0,7 et 1,3 MHz.

15. Procédé selon une quelconque selon les revendications 1 à 14, dans lequel le champ magnétique varié est appliqué sur des particules magnétiques peu de temps avant l'administration à la zone d'examen.

16. Procédé selon une quelconque de la revendication 1 à 14, dans lequel des particules magnétiques sont administrées à la zone d'examen dans un état aggloméré et dans lequel, dans seulement une partie de la zone d'examen, les particules sont séparées.

17. Procédé selon une quelconque selon les revendications 1 à 16, dans lequel la fréquence du champ magnétique varié est proche de la fréquence du champ magnétique d'imagerie et dans lequel l'exposition au champ magnétique varié et l'exposition au champ magnétique d'imagerie s'alternent.

18. Procédé selon la revendication 17, dans lequel la fréquence du champ magnétique varié est 0,8 à 1,2 fois la fréquence du champ magnétique d'imagerie.

19. Procédé selon une quelconque des revendications précédentes, **caractérisé en ce que** la particule magnétique est une particule multi-domaine ou mono-domaine qui peut être inversement magnétisée par rotation de Néel et/ou rotation brownienne.

20. Procédé selon une quelconque des revendications précédentes, **caractérisé en ce que** la particule magnétique est une particule magnétique dure ou souple multi-domaine.

21. Appareil pour déterminer la distribution spatiale de particules magnétiques dans une zone d'examen dans un objet d'examen, comprenant :
a) des moyens pour générer un champ magnétique avec une distribution spatiale de l'intensité de champ magnétique de sorte que la zone d'examen soit constituée d'une première sous-zone avec une intensité inférieure de champ magnétique où la magnétisation des particules magnétiques n'est pas saturée et une seconde sous-zone avec une intensité supérieure de champ magnétique où la magnétisation des particules magnétiques est saturée,
b) des moyens pour changer la localisation spatiale des deux sous-zones dans la zone d'examen de sorte que la magnétisation des particules change localement,
c) des moyens pour l'acquisition de signaux qui dépendent de la magnétisation dans la zone d'examen influencée par ce changement,
d) des moyens pour l'évaluation desdits signaux pour obtenir des informations sur la distribution spatiale des signaux dans la zone d'examen, et
e) des moyens pour imposer, dans la zone d'examen, plus particulièrement, dans au moins des parties de la première sous-zone avec une intensité inférieure de champ magnétiques, un champ magnétique varié pendant au moins une certaine partie du temps, plus particulièrement périodiquement ou en continu, afin de réduire ou empêcher l'agglomération des particules magnétiques.
